Europäisches Patentamt

European Patent Office

Office européen des brevets

(11) Publication number: **0 286 115 B1**

## (12) EUROPEAN PATENT SPECIFICATION

(45) Date of publication of patent specification: **19.08.92** (51) Int. Cl.⁵: **C12P 5/02**

(21) Application number: **88105624.6**

(22) Date of filing: **08.04.88**

(54) **A method for anaerobic fermentation of organic substances, especially for the production of "biogas".**

(30) Priority: **09.04.87 DK 1811/87**

(43) Date of publication of application:
**12.10.88 Bulletin 88/41**

(45) Publication of the grant of the patent:
**19.08.92 Bulletin 92/34**

(84) Designated Contracting States:
**AT BE CH DE ES FR GB GR IT LI LU NL SE**

(56) References cited:
**EP-A- 0 114 769**

**BIOMASS, vol. 2, 1982, pages 43-55, Applied Science Publishers Ltd., GB; D.A.STAFFORD: "The effects of mixing and volatile fatty acid concentrations on anaearobic digester performance"**

**JOURNAL OF THE AMERICAN OIL CHEMISTS SOCIETY, vol. 60, no. 2, February 1983, pages 314-315, Champaign, Illinois, US; J.T.L.ONG: "Oil recovery from spent bleaching earth and disposal of the extracted material"**

(73) Proprietor: **CH4 ENERGI A/S
Abildager 11
DK-2605 Brondby(DK)**

(72) Inventor: **Johansen, Kjeld
Trendavej 10
DK-9640 Farso(DK)**

(74) Representative: **Roerboel, Leif et al
BUDDE, SCHOU & CO. A/S Sundkrogsgade 10
DK-2100 Copenhagen O(DK)**

Rank Xerox (UK) Business Services

## Description

### TECHNICAL FIELD

The present invention relates to a method of the kind set forth in the preamble of claim 1.

### BACKGROUND ART

Such a method is known from the European patent application No. 0 114 769. In this application it is disclosed that the fatty acids or compounds or derivatives thereof are to be added in very small concentration, as there is otherwise a risk that they inhibit the reaction instead of supporting it. The application referred to proposes basing the additives on unsaturated fatty acids with between 18 and 22 carbon atoms and at least two unsaturated olefinic bonds per molecule in a concentration of between 0.001 and 0.35 mmol per litre of the process liquid. As examples of fatty acids used said application mentions linseed oil, linoleic acid or linolenic acid or an ester of the latter.

Said European application also mentions that the fatty acids shall either be soluble in the process liquid or made into an emulsion prior to being added to the process liquid.

It must be considered as a substantial drawback with the method thus known that it necessitates rather great care when adding the fatty acids and/or the compounds and/or derivatives thereof. It is a further drawback that these substances may be expensive to procure.

### DISCLOSURE OF THE INVENTION

It is the object of the present invention to provide a method of the kind initially referred to, that does not suffer from the drawbacks mentioned, and this object may be achieved by using a method, according to the present invention characterized by the method step set forth in the characterizing clause of claim 1. Using a carrier mass results in a substantial reduction of the risk of the concentration of the fatty acids or the compounds or derivatives thereof becoming too high, especially locally, and further, it is unnecessary to emulgate them before adding them to the process liquid.

It will be obvious that the production per se of a mixture as said forth in the characterizing clause of claim 1 in itself would not represent any saving in economy, but this problem is solved by proceeding as set forth in claim 2. This embodiment is based on the use of a waste material, which up to the present has been difficult to dispose of, and hence may be procured at very low cost. A typical example of such a mixture is bentonite, which has been used for the purification of edible oils and in an adsorbed condition contains various fatty acids etc., regarded as unwanted in a commercial product. Correspondingly, according to the invention one may proceed as set forth in claim 3.

The method of the present invention may be used with anaerobic fermentation of various organic substances, especially waste material, but in practice the method has especially proved suitable for processing the substances set forth in claim 4.

### EXAMPLE

The following example serves to illustrate how the method of the present invention may be carried out, without, however, imposing any limits on the scope of the invention as defined in the following claims.

During a period of one year, liquid manure (faeces mixed with urine) from a herd of milch cows was supplied to a biogas plant in an amount of approximately 5 metric tons per 24 hours.

The composition of the liquid manure comprised 6 to 8 per cent dry matter, during the conventional process yielding gas in a quantity of 100 cubic metres per 24 hours.

The addition per 24 hours of 150 kilogrammes bentonite containing approximately 30 to 40 per cent, i.e. approximately 45 to 60 kilogrammes, of adsorbed organic oily matter from the use of the bentonite for purifying edible oils caused an increase in the gas yield of 150 cubic metres per 24 hours, i.e. an increase of 150 per cent.

Of the increase in gas yield, only approximately 80 to 90 cubic metres per 24 hours can be ascribed to the oily matter added with the bentonite, so that the remainder can only be ascribed to an increased decomposition of the organic matter in the biogas plant. This corresponds to an observed substantial reduction of the amount of residual dry matter leaving the biogas plant.

Thus it has been observed that the total amount of organic dry matter is reduced by approximately 65 to 75 per cent when carrying out the method of the present invention, as compared to approximately 35 per cent with the conventional method not comprising the use of an additive containing organic fatty or oily matter.

As an experiment, the supply of liquid manure has been interrupted for a period, whereas the bentonite/oily matter additive has been added in approximately the same quantity as before. This has resulted in a reduction in the gas yield of more than 50 per cent after a period of 5 to 7 days. From this, it is reasonable to assume that the reduced gas yield is mainly produced from the oily matter added with the bentonite, together with a small amount resulting from the decomposition of the slight residue or organic dry matter not yet decomposed. After the resumption of the normal supply of liquid matter, the gas yield increases to normal in a few days.

## Claims

1. A method for anaerobic fermentation of organic substances, especially organic waste material for producing at least one hydrocarbon gas, such as methane, while using fatty acids and/or fatty acid compounds and/or fatty acid derivatives to accelerate the anaerobic process, **characterized** in that the fatty acids and/or the compounds and/or derivatives thereof are added as a component of a mixture, the main component of which consists of a carrier mass in the form of one or a number of substances with a large surface area in relation to the volume, whereas the fatty acids or the compounds or derivatives thereof are adsorbed on the surface of the elements of the carrier mass, said elements constituting small particles, fibres or other elements with a great surface area compared to the volume.

2. A method according to claim 1, **characterized** in that as the mixture mentioned is used such a one being the result of using the carrier mass for purifying vegetable or animal oils or fats by adsorbtion of unwanted fatty acids or fatty acid compounds or derivatives and subsequent separation of the purified oils or fats from the carrier mass with the substances adsorbed thereon.

3. A method according to claim 1 or claim 2, **characterized** in that as carrier mass there is used a very fine-grained mineral material, such as bentonite, fuller's earth or the like.

4. A method according to anyone or any of the claims 1 to 3, **characterized** in that as the organic substances to be subjected to anaerobic fermentation are used waste from animal stables, such as urine, manure and/or used litter.

## Patentansprüche

1. Verfahren zur anaeroben Fermentation von organischen Substanzen, insbesondere organischem Abfallmaterial, zur Herstellung von mindestens einem Kohlenwasserstoffgas, wie Methan, wobei Fettsäuren und/oder Fettsäureverbindungen und/oder Fettsäurederivate zur Beschleunigung des anaeroben Verfahrens verwendet werden, **dadurch gekennzeichnet,** daß die Fettsäuren und/oder Verbindungen und/oder Derivate davon als eine Komponente eines Gemisches hinzugegeben werden, dessen Hauptkomponente aus einer Trägermasse aus einer oder einer Anzahl von Substanzen mit einer großen Oberfläche im Verhältnis zum Volumen besteht, wobei die Fettsäuren oder die Verbindungen oder Derivate davon auf der Oberfläche der Elemente der Trägermasse adsorbiert sind, wobei die Elemente kleine Teilchen, Fasern oder andere Elemente mit einer großen Oberfläche verglichen mit dem Volumen bilden.

2. Verfahren nach Anspruch 1, **dadurch gekennzeichnet,** daß ein Gemisch verwendet wird, das das Resultat der Verwendung der Trägermasse zur Reinigung von pflanzlichen oder tierischen Ölen oder Fetten durch Adsorption von unerwünschten Fettsäuren, Fettsäureverbindungen oder Derivaten und nachfolgender Trennung der gereinigten Öle oder Fette von der Trägermasse mit den darauf adsorbierten Substanzen ist.

3. Verfahren nach Anspruch 1 oder 2, **dadurch gekennzeichnet,** daß als Trägermasse ein sehr feinkörniges minerales Material wie Bentonit, Fullererde oder ähnliches verwendet wird.

4. Verfahren nach einem der Ansprüche 1 bis 3, **dadurch gekennzeichnet,** daß als organische Substanzen, die anaerober Fermentation unterworfen werden sollen, Abfall aus Tierställen wie Urin, Kot und/oder gebrauchte Streu verwendet wird.

## Revendications

1. Procédé de fermentation anaérobie de substances organiques, en particulier de déchets organiques, pour produire au moins un gaz hydrocarboné tel que le méthane, avec utilisation d'acides gras et/ou de composés d'acides gras et/ou de dérivés d'acides gras pour accélérer le processus anaérobie, caractérisé en ce que les acides gras et/ou les composés et/ou dérivés de ceux-ci sont ajoutés en tant que composant d'un mélange dont le composant principal consiste en une masse de support sous forme d'une substance ou d'un certain nombre de substances de grande aire de surface par rapport au volume, les acides gras ou les composés ou dérivés de ceux-ci étant adsorbés sur la surface des éléments de la masse de support, lesdits éléments constituant de petites particules, fibres ou autres éléments de grande aire de surface par rapport au volume.

**2.** Procédé selon la revendication 1, caractérisé en ce que l'on utilise comme mélange mentionné un mélange qui résulte de l'utilisation de la masse de support pour purifier des huiles ou graisses végétales ou animales par adsorption des acides gras, composés d'acides gras ou dérivés d'acides gras indésirables et de la séparation des huiles ou graisses purifiées et de la masse de support sur laquelle sont adsorbées les substances.

**3.** Procédé selon la revendication 1 ou la revendication 2, caractérisé en ce que l'on utilise comme masse de support une matière minérale à grains très fins telle que la bentonite, la terre à foulon ou analogues.

**4.** Procédé selon l'une quelconque ou l'une des revendications 1 à 3, caractérisé en ce que l'on utilise comme substances organiques qui doivent être soumises à une fermentation anaérobie des déchets provenant d'étables d'animaux, tels que l'urine, le purin et/ou la litière usagée.